(19) Europäisches Patentamt
European Patent Office
Office européen des brevets

(11) Publication number: **0 422 904 A2**

# (12) EUROPEAN PATENT APPLICATION

(21) Application number: **90311065.8**

(51) Int. Cl.[5]: **G09G 3/36**

(22) Date of filing: **09.10.90**

(30) Priority: **09.10.89 JP 263556/89**

(43) Date of publication of application: **17.04.91 Bulletin 91/16**

(84) Designated Contracting States: **DE FR GB NL**

(71) Applicant: **SHARP KABUSHIKI KAISHA**
**22-22 Nagaike-cho Abeno-ku**
**Osaka 545(JP)**

(72) Inventor: **Koden, Mitsuhiro**
**Reinbou Manshyon Kawaramachi 401, 27-6**
**Nishikitsuji-cho, Nara-shi, Nara-ken(JP)**
Inventor: **Numao, Takaji**
**1-1-302, Saidaijikunimi-cho**
**Nara-shi, Nara-ken(JP)**

(74) Representative: **Brown, Kenneth Richard et al**
**R.G.C. Jenkins & Co. 26 Caxton Street**
**London SW1H 0RJ(GB)**

## (54) Method of driving antiferroelectric liquid crystal device.

(57) A method of driving a liquid crystal device having a substrate on which a plurality of scanning electrodes are arrayed in parallel to each other and a substrate on which a plurality of signal electrodes are arrayed in parallel to each other, the substrates being opposed so that the scanning electrodes and the signal electrodes are intersectionally placed to each other, and sandwiching an antiferroelectric liquid crystal phase between the substrates, comprising the steps of:
applying, at odd-numbered frames, a selection voltage whose average potential is positive followed by a non-selection voltage whose average potential is positive to each of the scanning electrodes, and applying, at even-numbered frames, a selection voltage whose average potential is negative followed by a non-selection voltage whose average potential is negative to each of the scanning electrodes, so as to control the tristable switching state of the antiferroelectric liquid crystal phase at a plurality of pixels defined by the intersection area of each of the scanning electrodes and each of the signal electrodes in combination with voltages applied to the each of the signal electrodes, thereby turning each pixel on or off to provide a predetermined image.

EP 0 422 904 A2

FIG. 2

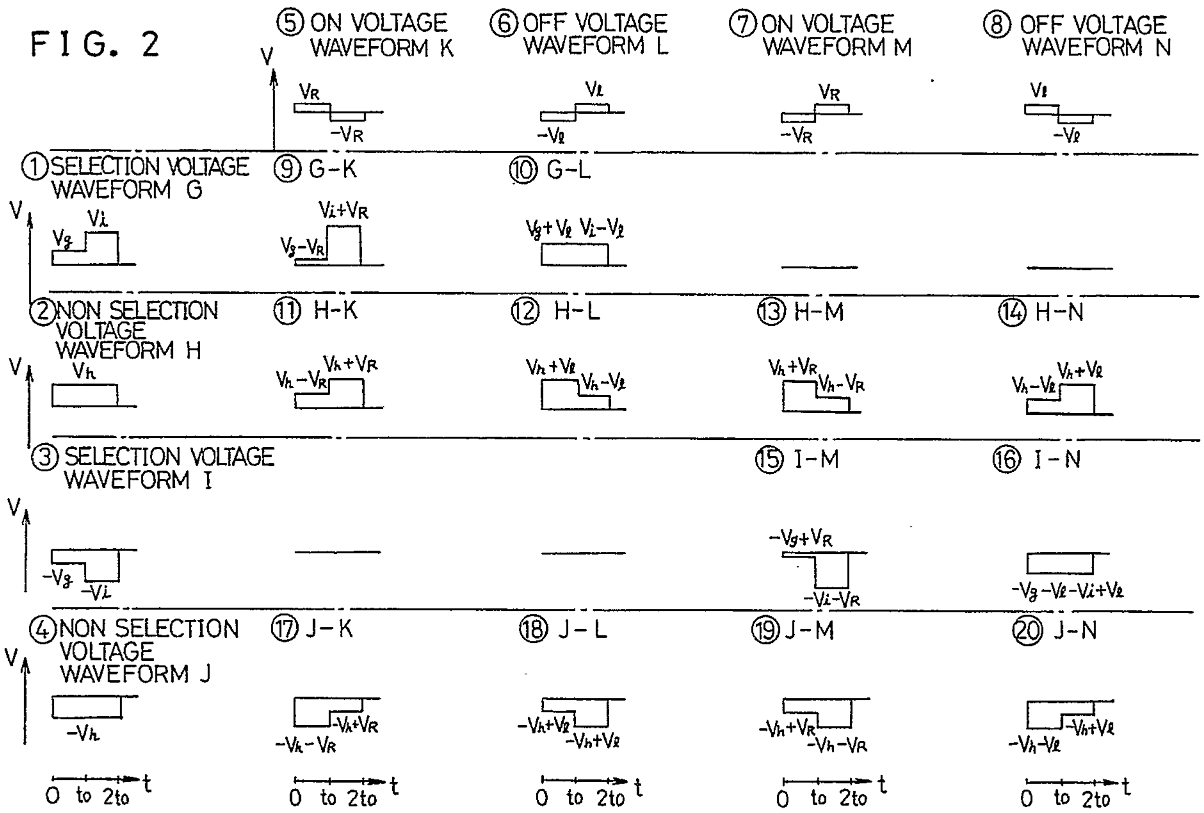


DRIVING WAVEFORM 1 IN ANTIFERROELECTRICS

# METHOD OF DRIVING ANTIFERROELECTRIC LIQUID CRYSTAL DEVICE

## BACKGROUND OF THE INVENTION

### 1. Field of the Invention

The present invention relates to a method of driving a liquid crystal device and, in particular, to a method of driving a liquid crystal device in which an anti-ferroelectric liquid crystal that shows tristable switching is used.

### 2. Description of the Related Art:

A ferroelectric liquid crystal device [N.A. Clark, et al., Appl. Phys. Lett., 36 , 899 (1980)] in which a chiral smectic C phase ferroelectric liquid crystal is used has recently been under active development. A display method in which this ferroelectric liquid crystal is used is wide in visual angle, and a large-capacity display of 1000 x 1000 lines or more is possible. Therefore, this method is thought to be very promising. However, there exist problems in that good orientation of liquid crystal molecules and memory property thereof required in this display method are difficult to realize in actual liquid crystal cells, and the liquid crystal cells are vulnerable to impact from outside. A lot of problems to be solved for practical use remains.

Meanwhile, a compound having a new liquid crystal phase that shows tristable switching on a side whose temperature is lower than the above chiral smectic C phase has recently been discovered. A study of a new display method using this compound has started [A.D.L. Chandani, et al., Jpn. J. Appl. Phys., 27 , L729 (1988)].

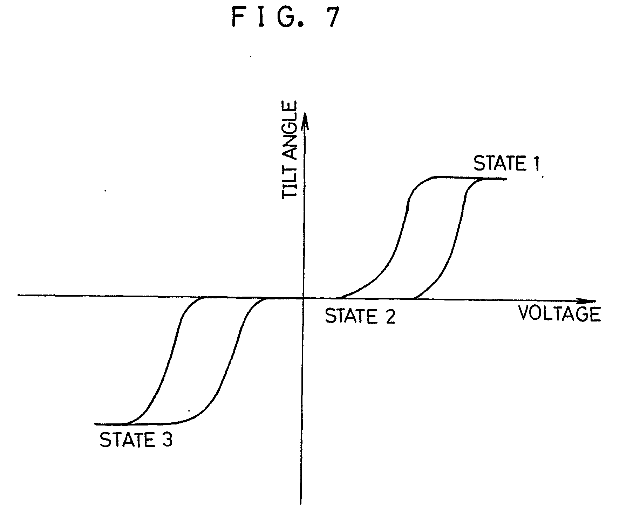

This new liquid crystal phase is still little known. Researchers use different ways of marking it. For example, it is expressed as the $S_Y^*$ phase (Japanese Patent Laid-Open No. Hei 1-213390) and the $SmC_A^*$ [Fukuda, the Japan Society for the Promotion of Science information science organic materials, 142th committee, 45th joint research materials, 34 (1989)]. This phase is said to be an antiferroelectric smectic phase the long axis of whose molecules is tilted with respect to the layer plane and having a helical structure. If the helices are disentangled by sealing into a liquid crystal cell thinner than the helical pitch length, the molecules are arrayed so that dipoles cancel each other layer by layer, as shown in Fig. 6(a). If an electric field is applied in this state, it is thought that it changes to the molecular configuration shown in Figs. 6(b) or 6(c) in which the dipoles are aligned in the direction of the voltage. The symbols ⊙ and ⊕ indicate the direction of the dipoles. Therefore, a bright and dark display can be made by, for example, combining polarizing plates. The relation between the applied voltage and the tilt angle is as shown in Fig. 7. It can take three stable states 1 to 3 and draws a hysteresis curb. Therefore, display driving can be performed with this relation.

Only a few compounds that show this liquid crystal phase have been reported. It is said that, for example, the following compound shows this liquid crystal phase [Japanese Patent Laid-Open No. Hei 1-213390; Y. Suzuki, et al., Proc. 2nd International Conference on Ferroelectric Liquid Crystals, P-106 (1989)].

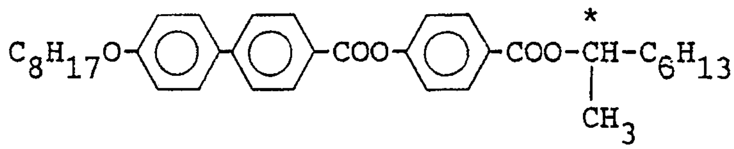

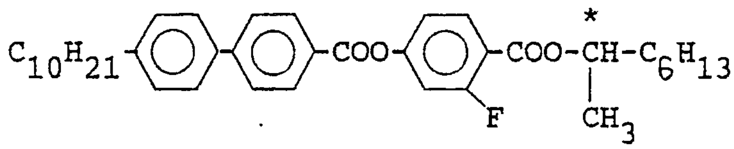

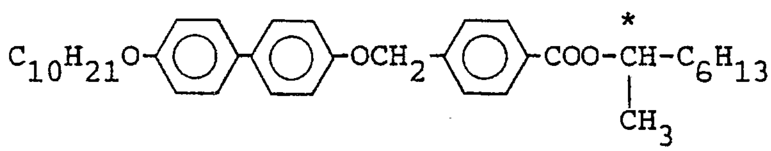

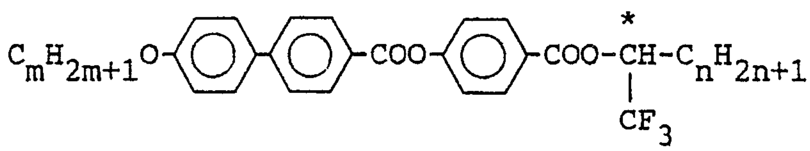

(n and m each denotes an integer of 6 to 12)

The tristable switching of the antiferroelectric liquid crystal phase is quite a new switching mode. So, at the present time, the research objectives center on the switching mechanism and the molecular configuration, and no matrix type display devices in which antiferroelectric liquid crystals are used have been reported. Of course, a matrix type display device itself can be produced if a transparent electrode is patterned as a matrix type by using ITO on a pair of substrates for the liquid crystal device and connected to the outside power source. However, such a matrix type liquid crystal device cannot become a good display device unless driving methods appropriate to its switching mode are combined. A large number of proposals [For example, e.g., Wakita, et al., National Technical Report, 33(1) , 44 (1987)] about a conventional method of driving a ferroelectric liquid crystal device have been reported. However, the method of driving a ferroelectric liquid crystal device in which bistable memory is used cannot be applied to an antiferroelectric liquid crystal device that shows stable tristable switching.

## SUMMARY OF THE INVENTION

The present invention has been accomplished in light of the above-described circumstances. An object of the present invention is to provide a method of driving a matrix type liquid crystal device in which an antiferroelectric liquid crystal phase that shows a new switching mode is used.

According to the present invention, there is provided a method of driving a liquid crystal device having a substrate on which a plurality of scanning electrodes are arrayed in parallel to each other and a substrate on which a plurality of signal electrodes are arrayed in parallel to each other, the substrates being opposed so that the scanning electrodes and the signal electrodes are intersectionally placed to each other, and sandwiching an antiferroelectric liquid crystal phase between the substrates, comprising the steps of:
applying, at odd-numbered frames, a selection voltage whose average potential is positive followed by a non-selection voltage whose average potential is positive to each of the scanning electrodes, and applying, at even-numbered frames, a selection voltage whose average potential is negative followed by a non-selection voltage whose average potential is negative to each of the scanning electrodes, so as to control the tristable switching state of the antiferroelectric liquid crystal phase at a plurality of pixels defined by the intersection area of each of the scanning electrodes and each of the signal electrodes in combination with voltages applied to each of the signal electrodes, thereby turning each pixel on or off to provide a predetermined image.

In the driving method of the present invention, since a specific voltage waveform of different patterns in the odd-numbered frames and in the even-numbered frames in a picture image frame cycle is applied to each scanning electrode, the control of the tristable states (states 1 to 3) regarding antiferroelectric liquid

crystals in each pixel can be made with ease. In particular, an applied waveform which is inverted in the odd- and even-numbered frames is provided. As a result, the case (for example, a bright state) where antiferroelectric liquid crystals in each pixel is switched at every frame between state 1 and state 3 and the case (for example, a dark state) where it is switched at every frame in state 2 can be selected, thereby keeping each pixel stably turned on or turned off.

## BRIEF DESCRIPTION OF THE DRAWINGS

Fig. 1 is a schematic view of a matrix type antiferroelectric liquid crystal device for explaining an embodiment of the present invention;
Figs. 2 to 4 are voltage waveform charts for explaining the method of driving an antiferroelectric liquid crystal device of the present invention;
Fig. 5 is a cross sectional view of an antiferroelectric liquid crystal device for explaining the embodiment of the present invention;
Fig. 6 is an explanatory view showing the molecular configuration in an antiferroelectric liquid crystal phase; and
Fig. 7 is an explanatory view showing the relation between the applied voltage and the tilt angle in the antiferroelectric liquid crystal phase.

## DETAILED DESCRIPTION OF THE PREFERRED EMBODIMENTS

The embodiments of the present invention will be explained hereinunder by taking a 16 x 16 simple matrix type liquid crystal device as an example. Fig. 1 is a schematic top plan view of a simple matrix liquid crystal device with 16 scanning electrodes $L_1$ to $L_g$ and 16 signal electrodes $S_1$ to $S_g$. These scanning electrodes are denoted as $L_1$, $L_2$, $L_3$ -$L_g$ in the order from the top; signal electrodes are denoted as $S_1$, $S_2$, $S_3$ - $S_g$ from the left. A region where each scanning electrode $L_i$ and each signal electrode $S_j$ overlap each other is denoted as a pixel $A_{ij}$ (i and j are each a positive integer). A scanning-side driver 10 is connected to the scanning electrodes of this simple matrix panel, and a signal-side driver 11 is connected to the signal electrodes.

This matrix type liquid crystal device is, for example, driven by the drive waveform shown in Fig. 2, as shown in Fig. 3. The driving method will now be explained in detail. In the odd-numbered frames, first, a selection voltage waveform G (a stepwise uneven waveform; the average potential is positive) of ① shown in Fig. 2 is applied in sequence to scanning electrodes $L_1$ to $L_g$. In the even-numbered frames, a selection voltage waveform I (the average potential is negative) of ③ is applied in sequence to scanning electrodes $L_1$ to $L_g$. After the selection voltage waveform G of ① has been applied to the scanning electrodes $L_i$, a non-selection voltage waveform H (an even waveform; the average potential is positive) of ② is applied to the scanning electrode $L_i$ in the same frame. After the selection voltage waveform I of ③ has been applied to the scanning electrode $L_i$, a non-selection voltage waveform J (the average potential is negative) of ④ is applied to the scanning electrode $L_1$ in the same frame.

In such a scanning state of the scanning electrodes, if it is desired that pixel $A_{ij}$ be placed in state 1 of Fig. 7 in an odd-numbered frame, an ON voltage waveform (whose average potential is zero) of ⑤ should be applied to a signal electrode $S_j$; if it is desired that pixel $A_{ij}$ be placed in state 2 of Fig. 7 in an odd-numbered frame, an OFF voltage waveform of ⑥ should be applied to a signal electrode $S_j$. In an even-numbered frame, if it is desired the pixel $A_{ij}$ be placed in state 3 of Fig. 7 in an even-numbered frame, an ON voltage waveform of ⑦ should be applied to a signal electrode $S_j$; if it is desired that pixel $A_{ij}$ be placed in state 2 of Fig. 7 in an even-numbered frame, an OFF voltage waveform of ⑧ should be applied to a signal electrode $S_j$. (At this time, the polarization axis of the polarizing plate is made to match with a direction perpendicular to the smectic layer in the state 2 of Fig. 7.) Voltage waveforms applied to a pixel of the intersection point of each scanning electrode and each signal electrode are shown in

⑨ to ⑳

of Fig. 2 when voltage waveforms ① to 4 described above are applied to scanning electrodes and voltage waveforms ⑤ to ⑧ are applied to signal electrodes in a matrix type liquid crystal device.

A pixel placed in state 1 by the voltage waveform ⑨ in an odd-numbered frame is placed in state 3 by a reverse voltage waveform

⑮

in the next even-numbered frame. This change of states (state 1 ↔ state 3) is repeated. The turned-on state is maintained during this period. Meanwhile, a pixel, for example, placed in state 2 by voltage waveforms

⑩ to ⑭.

is still placed in state 2 by reverse voltage waveforms to

⑯ to ⑳,

and a non-turned-on state is maintained. Therefore, a desired display can be made by selecting either of two such kinds of active, stable display states for each pixel.

Voltage waveforms applied to scanning electrodes $L_1$ and $L_f$, signal electrodes $S_1$ and $S_8$, and pixels $A_{1,1}$, $A_{1,8}$, $A_{f,1}$ and $A_{f,8}$ when a pattern "A" of Fig. 1 is displayed in the above way are shown in Figs. 3(A) and 3(B).

Drive waveforms of Fig. 4 may be used in place of the drive waveforms of Fig. 2. This driving method will now be explained in detail. In odd-numbered frames, a selection voltage waveform G (the average potential is positive) of ① shown in Fig. 2 is applied in sequence to scanning electrodes $L_1$ to $L_g$. In even-numbered frames, a selection voltage waveform I (the average potential is negative) of ③ is applied in sequence to scanning electrodes $L_1$ to $L_g$. After the selection voltage waveform G of ① has been applied to the scanning electrodes $L_i$, a non-selection voltage waveform H (the average potential is positive) of ② is applied to the scanning electrode $L_i$ in the same frame. After the selection voltage waveform I of ③ has been applied to the scanning electrodes $L_i$, a non-selection voltage waveform J (the average potential is negative) of ④ is applied to the scanning electrode $L_i$ in the same frame. If it is desired that pixel $A_{ij}$ be placed in state 1 of Fig. 7 in an odd-numbered frame, an ON voltage waveform of ⑤ should be applied to a signal electrode $S_j$; if it is desired that pixel $A_{ij}$ be placed in state 2 of Fig. 7 in an odd-numbered frame, an OFF voltage waveform of ⑥ should be applied to a signal electrode $S_j$. In an even-numbered frame, it if is desired that pixel $A_{ij}$ be placed in state 3 of Fig. 7 in an odd-numbered frame, an ON voltage waveform of ⑦ should be applied to a signal electrode $S_j$; it if is desired that pixel $A_{ij}$ be placed in state 2 of Fig. 7 in an odd-numbered frame, an OFF voltage waveform of ⑧ should be applied to a signal electrode $S_j$. (At this time, the polarization axis of the polarizing plate is made to match with a direction perpendicular to the smectic layer in state 2 of Fig. 7.) Voltage waveforms applied to a pixel of the intersection point of each scanning electrode and each signal electrode are shown in

⑨ to ⑳

when voltage waveforms ① to ④ described above are applied to scanning electrodes and voltage waveforms ⑤ to ⑧ are applied to signal electrodes in matrix type liquid crystal device.

Next, an antiferroelectric liquid crystal device to which the driving method of the present invention can be applied will be explained.

Fig. 5 is a cross sectional view illustrating an embodiment of the antiferroelectric liquid crystal device.

Fig. 5 shows an example of a transparent display device. Reference numeral 1 denotes an insulating substrate, 2 denotes a transparent electrode, 3 denotes an insulating film, 4 denotes an orientation control layer, 5 denotes a sealing agent, 6 denotes an antiferroelectric liquid crystal, and 7 denotes a polarizing plate.

As for the insulating substrate 1, a light-transmitting substrate, usually a glass substrate, is used. On the insulating substrate 1, transparent electrodes 2 of strip-like patterns, each made of electrically conductive thin films of $InO_3$, $SnO_2$, or ITO (Indium-Tin Oxide), are formed in a direction in which the electrodes 2 intersect each other. The transparent electrodes 2 are electrically connected to the above scanning-side driver 10 and the signal-side driver 11, and the above-mentioned drive voltage is applied.

The insulating film 3 is usually formed thereon. However, the film 3 may be omitted in some instances. As for the insulating film 3, for example, an inorganic thin film of $SiO_2$, $SiN_x$, $Al_2O_3$, etc., or an organic thin film of polyimide, photoresist resin, polymer liquid crystals, etc. may be used. Where the insulating film 3 is an inorganic thin film, it can be formed by deposition, sputtering, CVD (Chemical Vapor Deposition), or solvent application, etc. Where the insulating film 3 is an organic thin film, it can be formed by applying a solution in which an organic material is dissolved or its precursor solution with a spinner application method, an immersion application method, a screen printing method, a roll application method, etc. and curing it under predetermined curable conditions (heating, light irradiation, etc.), or by forming with deposition, sputtering, CVD, or a LB (Langumuir-Blodgett) method.

The orientation control layer 4 is formed on the insulating film 3. However, if the insulating film 3 is omitted, the orientation control layer 4 is directly formed on the electrically conductive film 2. As the orientation control layer, an inorganic layer is used in some instances and an organic layer is used in other instances.

Where an inorganic orientation control layer is used, as a method often used there is available an oblique deposition of silicon oxide. A rotational deposition method may also be used. Where an organic orientation control layer is used, nylon, polyvinyl alcohol, polyimide, etc. may be used, and a portion thereon is usually subjected to rubbing. The orientation control layer may be formed by using polymer liquid crystals, or LB films, or may be aligned by a magnetic field or a spacer edge method. A method in which $SiO_2$, or $SiN_x$ is formed by deposition, sputtering, and CVD, and rubbing thereon is performed is also possible.

Next, two substrates are opposedly placed together, and an antiferroelectric liquid crystal 6 is injected between the substrates to form a liquid crystal device. The polarizing plate 7 is disposed on the outer surface of the substrate in correspondence with the orientation direction of the liquid crystal molecules.

## EXAMPLE 1

An ITO film having a thickness of 1000Å and a pattern as shown in Fig. 1 is formed on two glass substrates, furthermore, an $SiO_2$ film having a thickness of 2000Å is formed, and a PVA film having a thickness of 300Å is applied, each of which is subjected to rubbing. Next, these tow glass substrates are opposedly placed to each other to provide a matrix electrode element at cell thickness of 2 µm with the same rubbing directions, and an antiferroelectric liquid crystal compound (I) is injected between the substrates.

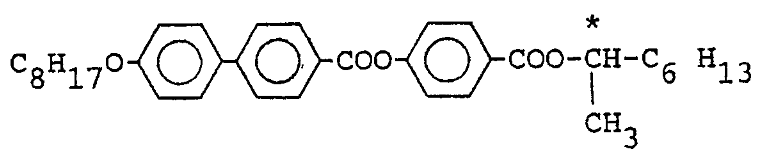

(I)

After the injection, the cells were once heated to a temperature at which the liquid crystal phase is transformed to an isotropic liquid. Thereafter, the cells were cooled to 70°C at a rate of 1°C/min., and an antiferroelectric liquid crystal device was obtained.

For the signal-side driver 11 of this liquid crystal device, SED-1600 produced by Seikosha Co. was used. For the scanning-side driver 10, a device formed by an analog switch and a shift register was used. When driving was performed using drive waveforms shown in Fig. 2 at 75°C, as shown in Fig. 3, a white and black matrix display could be performed. $V_g$, $V_i$, $V_l$, $V_h$ and $V_R$ was 5, 7, 1, 5, and 1 V, respectively, and $t_o$ was 300 msec.

## EXAMPLE 2

A matrix type antiferroelectric liquid crystal device was produced and driven in the same way except that the waveforms of Fig. 4 were used in place of the waveforms of

Fig. 2. A white and black matrix display could also be performed with these drive waveforms.

As has been explained above, a matrix display of a liquid crystal device in which antiferroelectric liquid crystals are used can be performed efficiently by the use of the driving method of the present invention.

Many different embodiments of the present invention can be made without departing from the spirit and scope thereof, therefore, it is to be understood that this invention is not limited to the specific embodiments thereof except as defined in the appended claims.

There are described above novel features which the skilled man will appreciate give rise to advantages. These are each independent aspects of the invention to be covered by the present application, irrespective of whether or not they are included within the scope of the following claims.

## Claims

1. A method of driving a liquid crystal device having a substrate on which a plurality of scanning electrodes are arrayed in parallel to each other and a substrate on which a plurality of signal electrodes are arrayed in parallel to each other, the substrates being opposed so that the scanning electrodes and the signal electrodes are intersectionally placed to each other, and sandwiching an antiferroelectric liquid crystal phase between the substrates, comprising the steps of:
applying, at odd-numbered frames, a selection voltage whose average potential is positive followed by a non-selection voltage whose average potential is positive to each of the scanning electrodes, and applying, at even-numbered frames, a selection voltage whose average potential is negative followed by a non-selection voltage whose average potential is negative to each of the scanning electrodes, so as to control the tristable switching state of the anti-ferroelectric liquid crystal phase at a plurality of pixels defined by the intersection area of each of the scanning electrodes and each of the signal electrodes in combination with voltages applied to the each of the signal electrodes, thereby turning each pixel on or off to provide a predetermined image.

2. The method of claim 1 in which the selection voltage applied at the odd- or even-numbered frames has a stepwise uneven waveform.

3. The method of claim 1 in which the non-selection voltage applied at the odd- or even-numbered frames has an even waveform.

4. The method of claim 1 in which the voltage applied to the signal electrodes has a rectangular waveform whose average potential is kept to be zero.

5. The method of claim 1 in which the antiferroelectric liquid crystal phase comprises at least one compounds of:

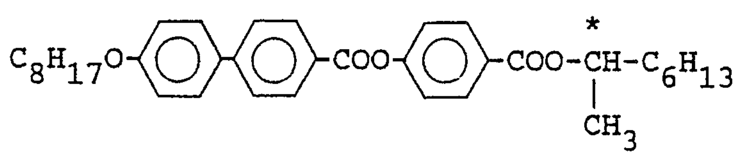

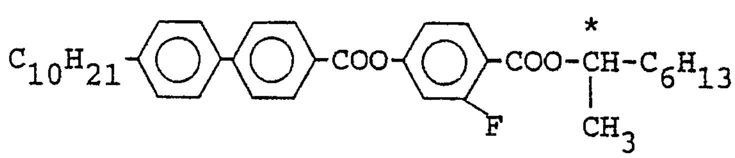

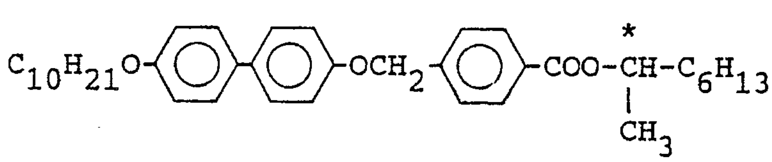

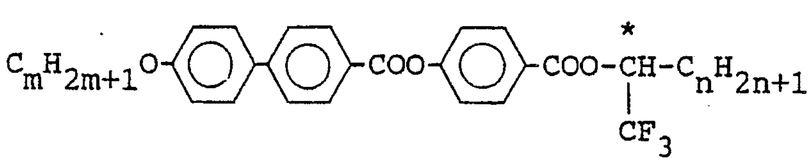

wherein n and m each denotes an integer of 6 to 12.

6. A method of driving a liquid crystal device having a row-and-column matrix of display pixels and electrodes formed on opposed substrates between which is disposed an antiferroelectric liquid crystal phase material, the method comprising applying to said electrodes drive signals which drive the device repetitively in a succession of frames to form a desired display in which each pixel is put in an on or off condition according to the voltage applied across the liquid crystal at the position of that pixel, wherein the voltages applied to the pixels are reversed from one frame to the next, said voltages being such that for one of said on and off conditions a pixel switches between two out of three stable states of the liquid crystal between successive frames and such that for the other of said on and off conditions a pixel stays in the third of said three stable states of the liquid crystal in successive frames.

F I G. 1

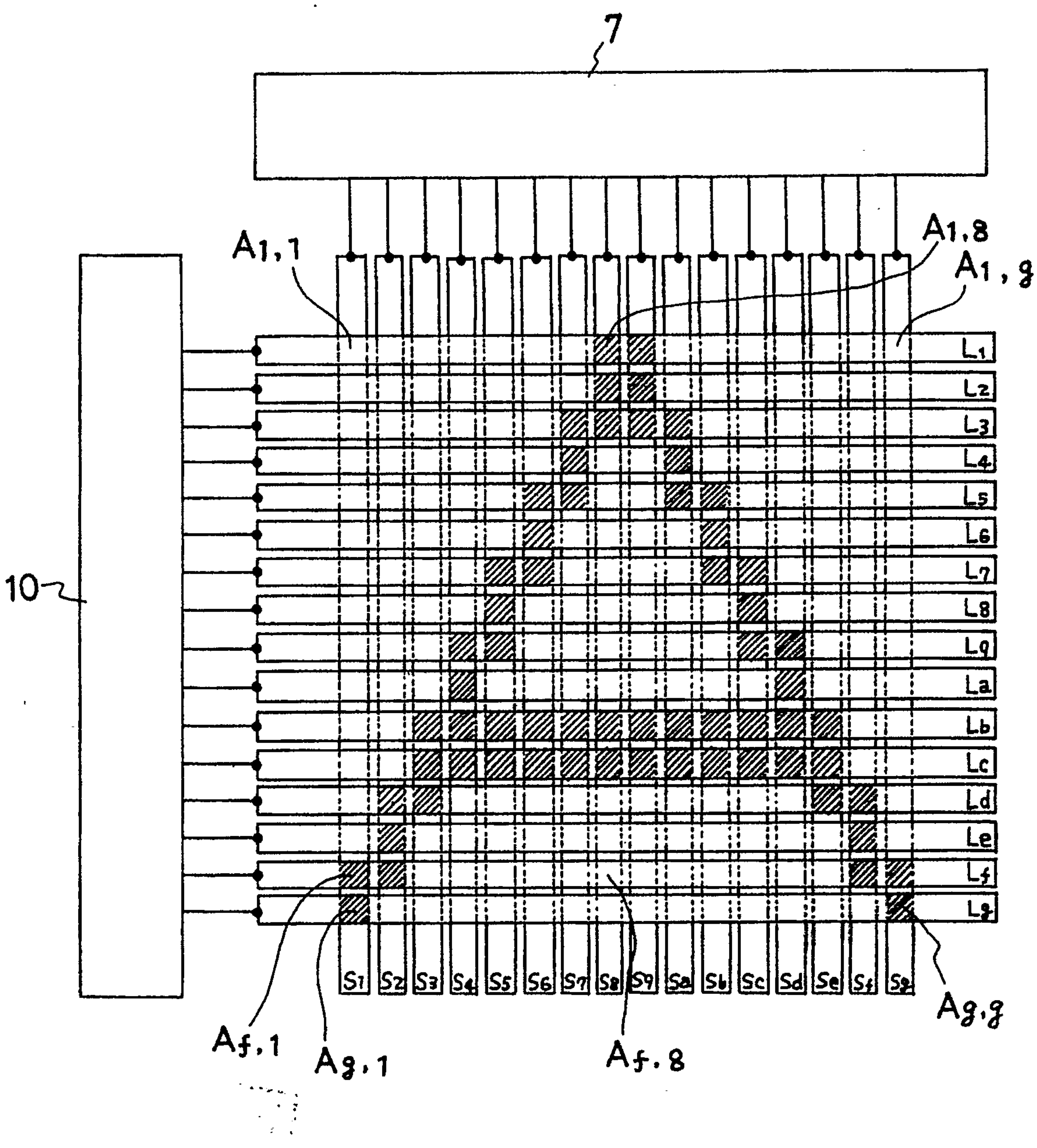
7
10
A1,1
A1,8
A1,g
L1
L2
L3
L4
L5
L6
L7
L8
L9
La
Lb
Lc
Ld
Le
Lf
Lg
S1
S2
S3
S4
S5
S6
S7
S8
S9
Sa
Sb
Sc
Sd
Se
Sf
Sg
Af,1
Ag,1
Af,8
Ag,g

FIG. 2

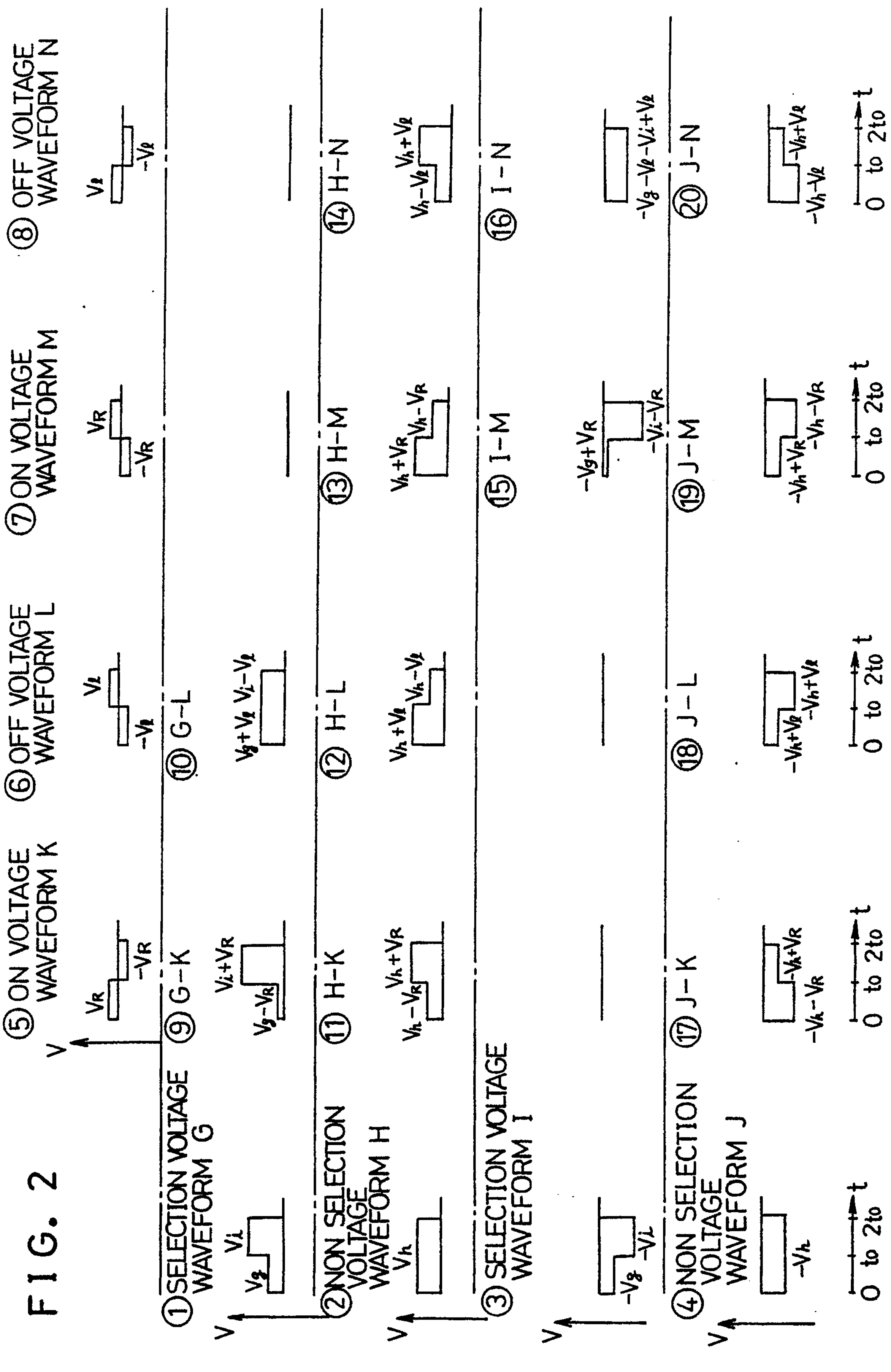


DRIVING WAVEFORM 1 IN ANTIFERROELECTRICS

FIG. 3(A)

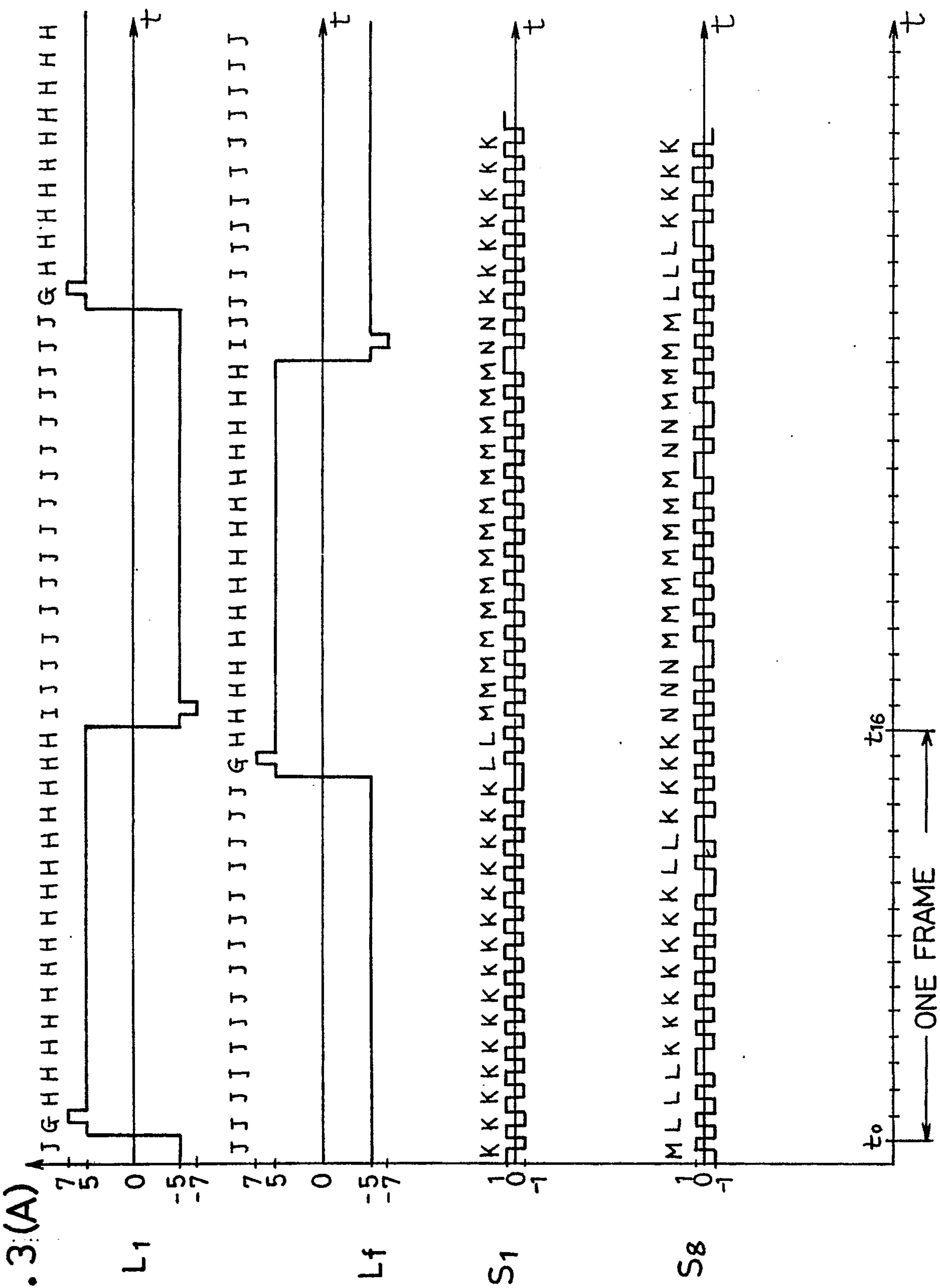

FIG. 3(B)

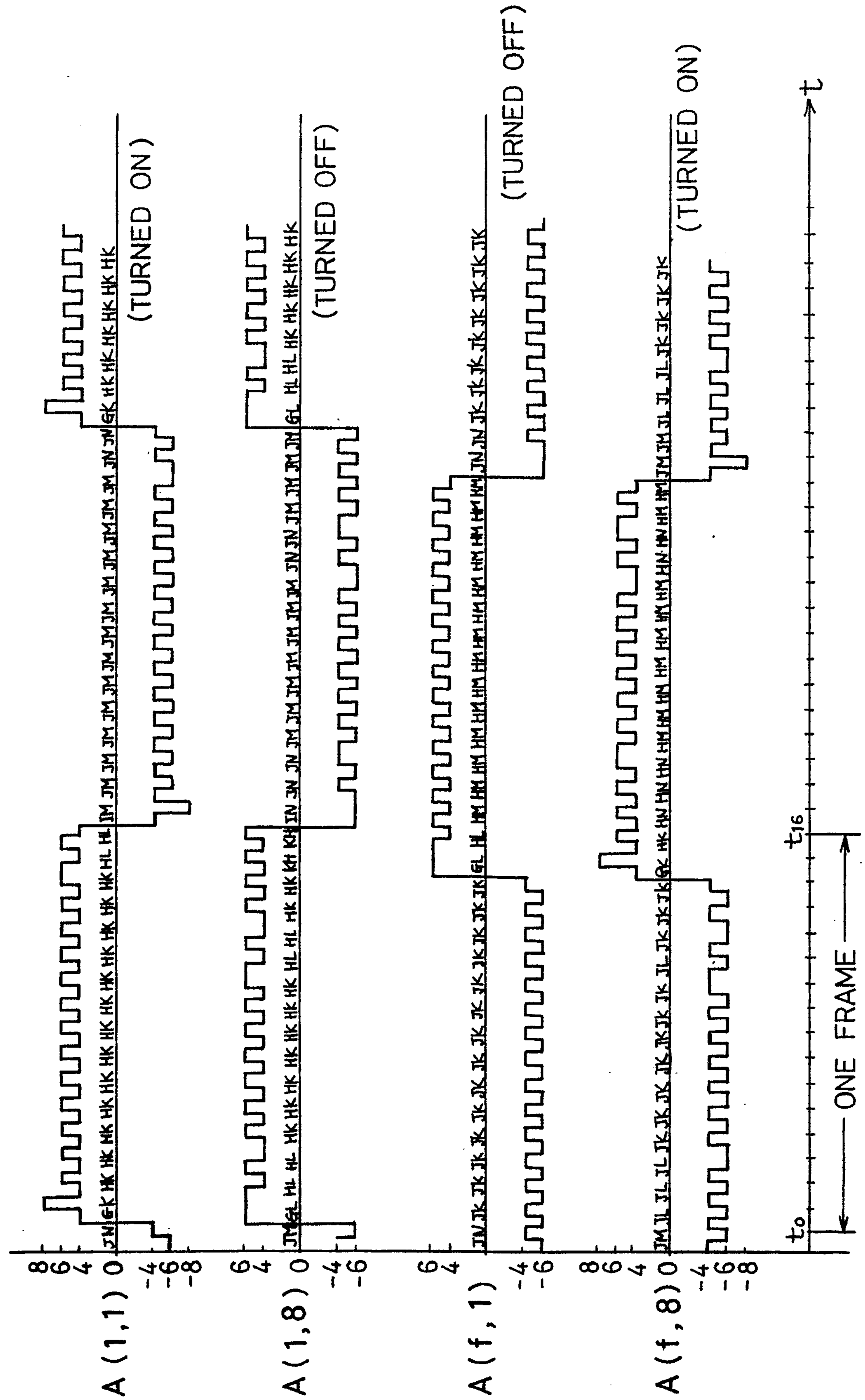
A(1,1)
(TURNED ON)
A(1,8)
(TURNED OFF)
A(f,1)
(TURNED OFF)
A(f,8)
(TURNED ON)
$t_0$
$t_{16}$
ONE FRAME
t

FIG. 4

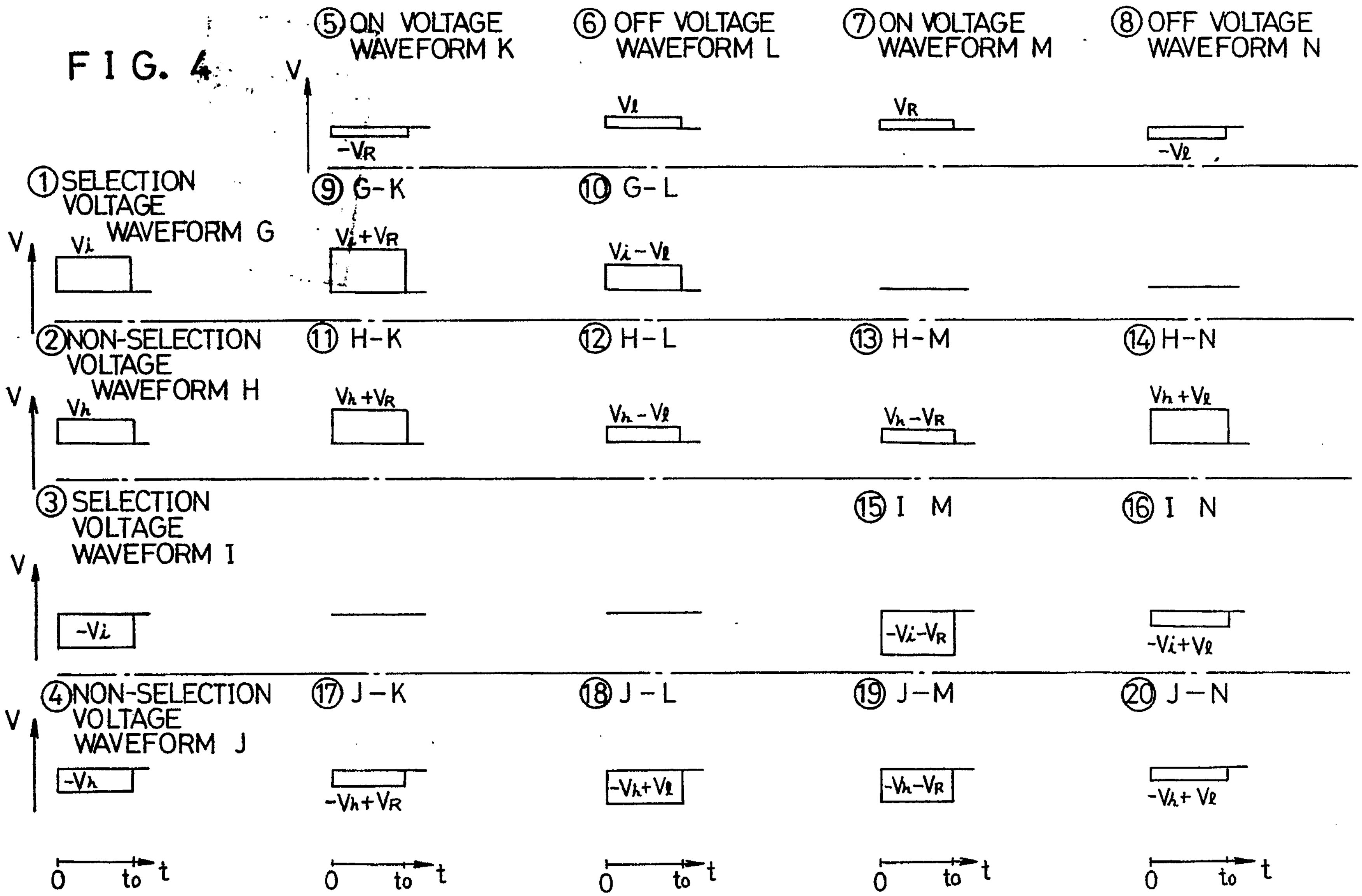


DRIVING WAVEFORM 2 IN ANTIFERROELECTRICS

FIG. 5

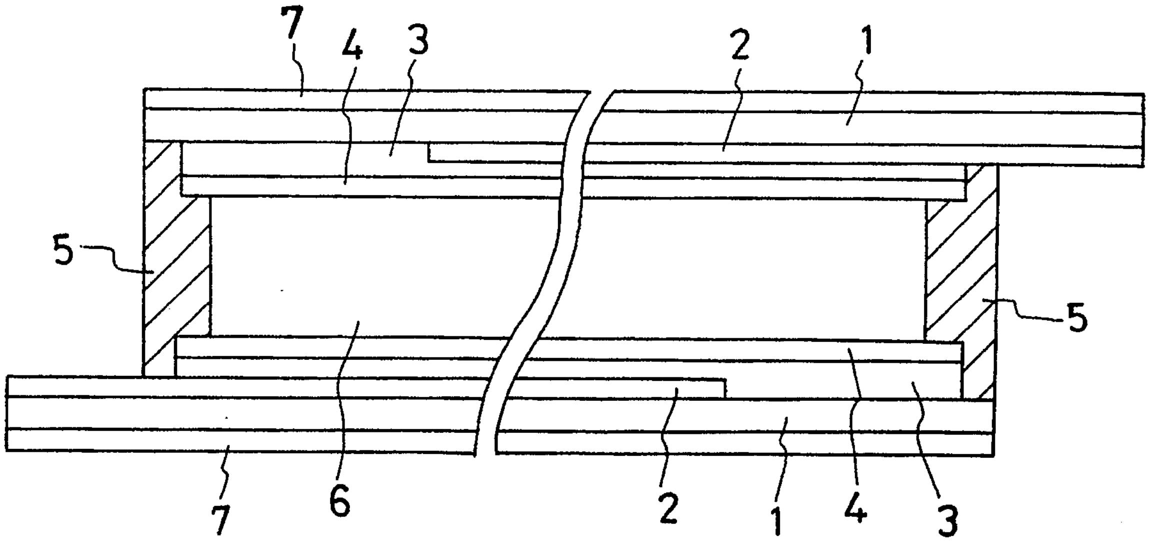
7
4
3
2
1
5
5
7
6
2
1
4
3

FIG. 6

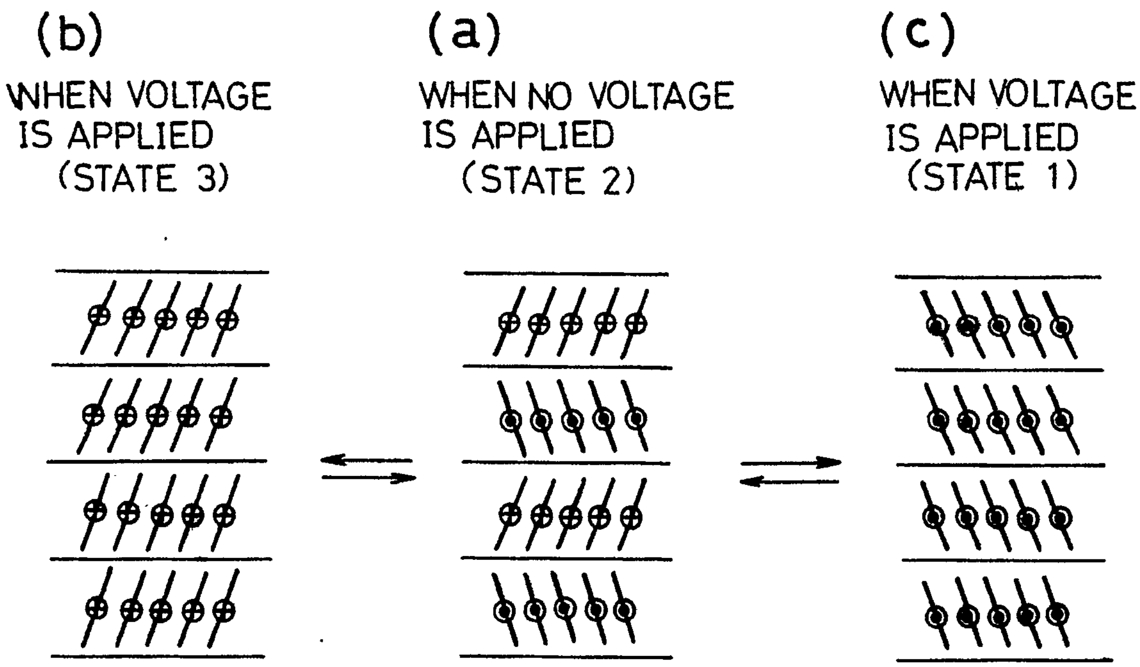
(b)
WHEN VOLTAGE IS APPLIED (STATE 3)
(a)
WHEN NO VOLTAGE IS APPLIED (STATE 2)
(c)
WHEN VOLTAGE IS APPLIED (STATE 1)

FIG. 7